# EUROPÄISCHE PATENTSCHRIFT

(11) **EP 1 957 442 B1**
(45) Veröffentlichungstag und Bekanntmachung des Hinweises auf die Patenterteilung: **11.11.2015**
(21) Anmeldenummer: 06819720.1
(22) Anmeldetag: 23.11.2006
(51) Int. Cl.: C07C 303/24, C07C 305/06

(54) **VERFAHREN ZUR HERSTELLUNG VON SCHWEFELSÄUREMONOESTERN AUS AMINOALKANOLEN**
PROCESS FOR PREPARING SULFURIC MONOESTERS FROM AMINO ALKANOLS
PROCEDE DE FABRICATION DE MONOESTERS D'ACIDE SULFURIQUE A PARTIR D'AMINOALCANOLS

(30) Priorität: 02.12.2005 DE 102005057897
(43) Veröffentlichungstag der Anmeldung: 20.08.2008
(73) Patentinhaber: BASF SE, 67056 Ludwigshafen (DE)
(72) Erfinder: DUPUIS, Jacques, 68775 Ketsch (DE); HOFFMANN, Reinhard, 67374 Hanhofen (DE); WINKLER, Ekhard, 67112 Mutterstadt (DE); WINTER, Manfred, 67596 Dittelsheim-Hessloch (DE)
(74) Vertreter: Peatfield, Jeremy William
(86) Internationale Anmeldenummer: PCT/EP2006/068842
(87) Internationale Veröffentlichungsnummer: WO 2007/063032

(56) Entgegenhaltungen:
- DE-A1- 2 840 554
- DE-A1- 10 124 300

## Beschreibung

Die Erfindung betrifft ein Verfahren zur Herstellung von Schwefelsäuremonoestern aus Aminoalkanolen durch Reaktion von Schwefelsäure mit Aminoalkanolen und Entfernen des bei der Reaktion entstehenden Wassers aus dem Reaktionsgemisch.

Schwefelsäuremonoester von Aminoalkanolen sind Zwischenprodukte. So wird beispielsweise der Schwefelsäuremonoester von 2-Aminoethanol als Ausgangsprodukt bei der großtechnischen Herstellung von Ethylenimin verwendet. Schwefelsäuremonoester von Aminoalkanolen werden nach dem Verfahren der DE-A 28 40 554 durch gleichzeitige Zugabe von Aminoalkanolen und Schwefelsäure zu einem im Bereich von 70 bis 150°C siedenden Suspensionsmittel wie Oktan, unter Durchmischung, Abdestillieren des bei der Reaktion entstehenden sowie gegebenenfalls mit den Ausgangstoffen zugeführten Wassers zusammen mit einem Teil des Suspensionsmittels als Azeotrop, Abtrennen des Wassers und Rückführen des Suspensionsmittels in die Reaktionsmischung hergestellt, wobei man das abdestillierte Suspensionsmittel durch frisches Suspensionsmittel ersetzt, das gasförmig beispielsweise mit einer Temperatur von 110 bis 200°C in die Reaktionsmischung eingebracht wird. Das Verfahren hat mehrere Nachteile. Es erfordert z. B. einen hohen Energieaufwand sowie einen großen apparativen Aufwand für die Lagerung und Aufarbeitung des Suspensionsmittels. Außerdem müssen die Kessel in bestimmten Abständen gereinigt werden, um Verbackungen von Schwefelsäuremonoestern zu entfernen.

Aus der DE-A 101 24 300 ist ein Verfahren zur Herstellung von Schwefelsäurehalbestern von Aminoalkanolen bekannt, wobei man Schwefelsäure und Aminoalkanole in Abwesenheit üblicher Suspensionsmittel in einer Wirbelschicht reagieren lässt und Wasser aus dem Reaktionsgemisch abdestilliert. Die Temperatur in der Wirbelschicht beträgt meistens 140 bis 160°C, kann jedoch auch bis zu 250°C betragen. Die dafür in Betracht kommenden Apparaturen sind aufwendig und teuer.

Der Erfindung liegt die Aufgabe zugrunde, ein verbessertes Verfahren zur Herstellung von Schwefelsäuremonoestern aus Aminoalkanolen zur Verfügung zu stellen, das gegenüber den bekannten Verfahren einen geringeren Energiebedarf aufweist und eine höhere Raum-Zeit-Ausbeute liefert.

Die Aufgabe wird erfindungsgemäß gelöst mit einem Verfahren zur Herstellung von Schwefelsäuremonoestern aus Aminoalkanolen gemäß Anspruch 1 durch Reaktion von Schwefelsäure mit Aminoalkanolen und Entfernen des bei der Reaktion entstehenden Wassers aus dem Reaktionsgemisch, wenn man Schwefelsäure und mindestens ein Aminoalkanol mischt, die entstehende heiße Reaktionsmischung unter turbulenter Strömung in einen Behälter leitet und darin mit einem Fluid quencht. Bei dem Fluid handelt es sich um eine wässrige Base.

Das erfindungsgemäße Verfahren wird vorzugsweise kontinuierlich durchgeführt. Verdünnungsmittel bzw. Suspensionsmittel werden bei dem erfindungsgemäßen Verfahren nicht benötigt. Dadurch ergeben sich Vorteile bei der Aufarbeitung der Schwefelsäuremonoester. Die Konzentration der bei dem Verfahren eingesetzten Schwefelsäure beträgt beispielsweise 90 bis 100 Gew.-%, meistens 96 bis 98 Gew.-%. Man kann jedoch auch Schwefelsäure einsetzen, die überschüssiges Schwefeltrioxid enthält, sog. rauchende Schwefelsäure, oder die Veresterung der Aminoalkanole mit Schwefeltrioxid vornehmen.

Nach dem erfindungsgemäßen Verfahren können alle Aminoalkanole mit Schwefelsäure bzw. Schwefeltrioxid verestert werden. Aminoalkanole können beispielsweise mit Hilfe der folgenden Formel charakterisiert werden: 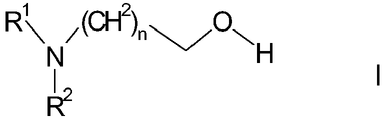 in der die Substituenten R¹ und R² gleich oder verschieden sind und für Wasserstoff, aliphatische Substituenten, die geradkettig, verzweigt und/oder cyclisch sein können und 1 bis 10 Kohlenstoffatome aufweisen, substituierte aliphatische Reste, deren Substituenten unter den Reaktionsbedingungen inert sind, und heterocycloaliphatische Reste mit 3 bis 6 Kohlenstoffatomen stehen, und n = 1 bis 10, vorzugsweise 1 bis 3, besonders bevorzugt n = 2 bedeutet.

Beispiele für solche Aminoalkohole sind Ethanolamine wie Monoethanolamin (2-Aminoethanol), Diethanolamin und Triethanolamin, Isopropanolamin, N-Isopropylethanolamin, N-Cyclohexylethanolamin, N-Cyclooctylethanolamin, 3-Aminopropanol und N-Cyclooctylisopropanolamin. Man kann auch Mischungen von Aminoalkoholen einsetzen. Besonders bevorzugt ist der Einsatz von Monoethanolamin als Aminoalkohol.

Das Molverhältnis von Schwefelsäure zu Aminoalkanol beträgt beispielsweise 1 : 1,5 bis 1 : 0,5, vorzugsweise 1 : 1 bis 1 : 0,9. In den meisten Fällen bringt man die beiden Reaktionspartner in äquimolaren Mengen zur Reaktion. Die Temperatur der Ausgangsstoffe, die miteinander zur Reaktion gebracht werden, entspricht meistens der Umgebungstemperatur. Sie liegt z.B. in dem Bereich von 5 bis 30°C, vorzugsweise 15 bis 25°C. Schwefelsäure und mindestens ein Aminoalkanol werden in einer Mischzone zusammengeführt, beispielsweise in einer Mischkammer oder mit Hilfe einer Mehrstoffdüse gemischt. Dabei tritt eine spontane Reaktion ein, die zu einer starken Temperaturerhöhung des Reaktionsgemisches führt. Die Temperaturerhöhung gegenüber der Temperatur der Reaktionsteilnehmer beträgt mindestens 200°C, beispielsweise 250 bis 300°C.

Man kann beispielsweise so verfahren, dass man Schwefelsäure und mindestens einen Aminoalkohol getrennt voneinander entweder parallel, gegeneinander oder unter einem beliebigen Winkel, vorzugsweise 30 bis 90° in eine Mischkammer pumpt. Beide Komponenten können jedoch auch mit Hilfe einer Mehrstoffdüse beispielsweise einer Zweistoffdüse, in einer Mischzone bzw. einer Mischkammer zusammengeführt werden. Aufgrund der stark exothermen Reaktion tritt eine sehr starke Temperaturerhöhung ein, die dazu führt, dass das Reaktionsprodukt in flüssiger Form anfällt und dass das bei der Veresterung entstehende Wasser spontan verdampft. Die Temperatur in der Reaktionszone beträgt mindestens 220°C, vorzugsweise mehr als 250°C. Beispielsweise liegt sie in dem Bereich von 260 bis 300°C. Sie kann z. B. geregelt werden, indem man ein Fluid in die Mischzone dosiert oder die Mischzone mit Hilfe eines Wärmeübertragungsmittels kühlt oder beheizt. Eine weitere Möglichkeit der Temperaturregelung besteht darin, dass man einen oder mehrere Zuläufe vorheizt bzw. abkühlt.

Das Reaktionsgemisch wird aus der Reaktionszone durch mindestens ein Rohr in einen Behälter geleitet, in dem es gequencht (d.h., mit einem kalten Fluid abgeschreckt) wird, um den bei der Reaktion entstandenen Wasserdampf zu kondensieren und je nach Bedarf die Schmelze kristallisiert oder in einem Lösemittel wie Wasser oder wässriger Base löst. Der Durchmesser des Rohres wird dabei so ausgelegt, dass das Reaktionsgemisch unter turbulenter Strömung in den nachgeschalteten Behälter geleitet wird. Die turbulente Strömung sorgt für eine weitere intensive Vermischung der Reaktionsteilnehmer. Beispielsweise kann man die Reaktionsmischung durch ein Rohr mit einem Durchmesser von mindestens 0,5 mm, z. B. Durchmesser in dem Bereich von 0,5 bis 1000 mm, vorzugsweise 1 bis 200 mm, in einen Behälter leiten und darin das Reaktionsgemisch mit einem Fluid, vorzugsweise einem wässrigen Medium quenchen. Das Rohr bzw. das Rohrbündel kann gerade oder auch wendelförmig beschaffen sein. Das Rohr bzw. Rohrbündel kann von einem Wärmeübertragungsmedium umgeben sein, so dass es möglich ist, dem Reaktionsgemisch Wärme zu entziehen oder zuzuführen. Auch eine direkte elektrische Beheizung ist möglich.

In einer bevorzugten Ausführungsform der Erfindung hat das Rohr am Anfang, d.h. am Abgang von der Mischkammer zum Quenchbehälter, einen kleineren Durchmesser als am Rohrende, das in den Quenchbehälter mündet. Beispielsweise beträgt der Rohrdurchmesser einer Laboratoriumsapparatur am Anfang 1 mm und am Ende des Rohres 5 mm. Der Übergang zu einem größeren Durchmesser kann kontinuierlich oder auch abschnittsweise in Segmenten erfolgen, deren Durchmesser beispielsweise jeweils um 1 mm zunimmt. Von besonderem Vorteil ist eine Anordnung, bei der ein Rohr, dessen Querschnitt sich von oben nach unten erweitert, wendelförmig ausgebildet ist.

In den wendelförmig gestalteten Rohren kommt es zu einer besonders intensiven Vermischung der Reaktionsteilnehmer. Aufgrund des bei der Reaktion entstehenden Wassers, das spontan verdampft, gelangt das Reaktionsgemisch mit sehr hoher Geschwindigkeit in den Quenchbehälter. Die Geschwindigkeit beträgt z. B. mindestens 10 m/s, beispielsweise 80 bis 240 m/s und maximal bis ca. 500 m/s.

In einer weiteren Ausgestaltung des erfindungsgemäßen Verfahrens ist das Rohr, das von der Mischkammer zum Quenchbehälter führt, so ausgestaltet, dass es an der Einmündung in den Quenchbehälter einen Durchmesser aufweist, der um den Faktor 2 bis 1000, vorzugsweise 10 bis 200 größer ist als am Ausgang der Mischkammer. In einer anderen Ausgestaltung der Erfindung wird das Reaktionsgemisch zunächst durch ein Rohr in einen Behälter geführt, indem es entspannt wird und gelangt danach in den Quenchbehälter. Das Rohr, das von der Mischkammer zum Entspannungsbehälter führt, kann einen konstanten Durchmesser aufweisen oder sich zum Entspannungsbehälter hin erweitern, wie oben beschrieben. Es kann gerade oder vorzugsweise wendelförmig ausgebildet sein.

Die heiße Reaktionsmischung wird nach dem Verlassen des Rohres vorzugsweise mit einem kalten Fluid gequencht. Zum Quenchen wird eine wässrige Base verwendet.

Die Temperatur der Quenchflüssigkeit kann beispielsweise -10 bis 80°C, insbesondere 20 bis 50°C betragen. Die Quenchflüssigkeit wird üblicherweise auf die aus dem Rohr bzw. Rohrbündel austretende Reaktionsmischung gesprüht, damit der Wasserdampf möglichst schnell kondensiert wird. Die Schwefelsäuremonoester von Aminoalkanolen sind unter den Reaktionsbedingungen flüssig. Sie fallen in Form von Tropfen in den Quechbehälter. Als Quechflüssigkeit kann man wässrige Lösungen von Natronlauge, Kalilauge oder einer anderen Base verwenden. Man erhält dann die entsprechenden Salze von Schwefelsäuremonoestern, die zum Beispiel für die Synthese von Ethylenimin benötigt werden.

Bei einem kontinuierlichen Betrieb der Anlage wird auch die Quenchflüssigkeit kontinuierlich in den Quenchbehälter geführt. Um beispielsweise die Natriumsalze von Schwefelsäuremonoestern von Aminoalkanolen in einer kontinuierlichen Arbeitsweise herzustellen, dosiert man kontinuierlich eine verdünnte wässrige Natronlauge in den Quenchkreislauf vor dem Quenchen der Reaktionsmischung und entnimmt dem Quenchkreislauf nach dem Verlassen des Quenchbehälters eine wässrige Lösung des Natiumsalzes von Schwefelsäuremonoester von Aminoalkanolen. Die Menge an wässriger Natronlauge, die dem Quenchkreislauf zugeführt wird, kann mit Hilfe des pH-Wertes der Quenchflüssigkeit geregelt werden. Die Quenchflüssigkeit enthält dann im stationären Zustand außer Natronlauge noch das Natriumsalz des Schwefelsäuremonoesters der jeweils eingesetzten Aminoalkanole in gelöster Form.

Besonders bevorzugt ist eine Verfahrensvariante, bei der die Umsetzung von Schwefelsäure mit Aminoalkanolen unter vermindertem Druck durchgeführt wird, beispielsweise bei Absolutdrücken von 1 bis 200 mbar, vorzugsweise 15 bis 50 mbar. Diese Angaben für den Druck beziehen sich auf den Druck im Quenchbehälter. Die Aufarbeitung des Reaktionsgemisches kann auch in einer Kaskade aus zwei oder mehreren Quenchbehältern vorgenommen werden. So kann man die Reaktionsmischung im ersten Behälter zunächst mit reinem Wasser quenchen und danach in einem zweiten Behälter die Neutralisation mit einer Base vornehmen. Eine weitere Variante bei der Aufarbeitung des Reaktionsgemisches besteht darin, dass man den Wasserdampf zunächst separat kondensiert und das flüssige Reaktionsprodukt (Schwefelsäuremonoester) anschließend auf einer Schuppenwalze oder einem Kühlband kristallisiert und es als Feststoff gewinnt.

Die Umsetzung kann beispielsweise in emaillierten Vorrichtungen, Keramik oder in Apparaturen aus Glas bzw. Quarzglas durchgeführt werden. Der besondere Vorteil des erfindungsgemäßen Verfahrens gegenüber den bekannten Verfahren liegt in der enormen Erhöhung der Raum-Zeit-Ausbeute, dem sehr niedrigen Energieverbrauch und darin, dass man die Reaktion in Abwesenheit von Verdünnungsmitteln wie einem Suspensionsmittel durchführen kann. Außerdem gibt es bei dem erfindungsgemäßen Verfahren keine apparatebedingte Probleme mit Abrasion und Verbackungen (Produktanbackungen an in den Apparaturen).

Schwefelsäuremonoester von Aminoalkanolen sind beispielsweise Zwischenprodukte für die Herstellung von Ethylenimin, das unter anderem zu Polyethylenimin verarbeitet wird. Polyethylenimin wird beispielsweise zur Verbesserung der Nassfestigkeit von Papier oder als Retentionsmittel bei der Papierherstellung verwendet.

Die Prozentangaben in den Beispielen bedeuten Gewichtsprozent.

### Beispiel 1

Am Kopf einer Mischkammer aus Glas waren zwei Kapillaren mit einem Durchmesser von 0,5 mm in einem Winkel von ca. 90° angeordnet. Am Boden der Mischkammer befand sich ein Rohr, das an dieser Stelle einen Durchmesser von 1 mm hatte und sich zum Ende hin in fünf Schritten auf 5 mm erweiterte. Das Ende dieses Rohres mündete am Kopf eines Quenchbehälters aus Glas. Hier befanden sich auch die Düsen, durch die die Quenchflüssigkeit in den Behälter gepumpt wurde. Das Rohr hatte eine Länge von insgesamt 120 cm. Es war wendelförmig angeordnet. Mischkammer und Wendelrohr waren ummantelt und befanden sich in einem Wärmeübertragungsmedium (Öl-bad), das eine Temperatur von 270°C hatte. Der Druck im Quenchbehälter wurde auf 36 mbar eingestellt.

Durch eine Kapillare wurden kontinuierlich 4,8 ml/min 96%ige Schwefelsäure und durch die andere Kapillare 5 ml/min Monoethanolamin (2-Aminoethanol) in die Mischkammer gepumpt. Die Temperatur der beiden Einsatzstoffe betrug vor dem Mischen 20°C. Die Temperatur des Reaktionsgemisches lag bei 270°C. Am unteren Ende des Rohres tropfte 2-Aminoethylhydrogensulfat (=Schwefelsäuremonoester von 2-Aminoethanol) in den Quenchbehälter, in dem die heiße Reaktionsmischung mit einer 10%igen wässrigen Natronlauge gequencht wurde. Nachdem der stationäre Zustand erreicht war, enthielt die Quenchflüssigkeit das Na-Salz von 2-Aminoethylhydrogensulfat und Natronlauge. Dann wurde Natronlauge pH-geregelt zur Quenchflüssigkeit vor Eingang in den Quechbehälter gepumpt, so dass die Quenchflüssigkeit einen pH-Wert von 10,7 hatte. Aus dem Sumpf des Quenchbehälters wurde kontinuierlich wässrige Lösung des Na-Salzes von 2-Aminoethylhydrogensulfat entnommen, indem man die Höhe der Quenchflüssigkeit im Quenchbehälter konstant hielt. Die Ausbeute betrug 98,9%.

### Beispiel 2

Beispiel 1 wurde mit folgenden Änderungen wiederholt: Die Menge an 96%iger Schwefelsäure betrug 4,8 ml/min, die Menge an Monoethanolamin 5 ml/min. Die Umsetzung wurde bei Normaldruck durchgeführt und der pH-Wert der Quenchflüssigkeit auf 9,9 eingestellt. Die Ausbeute betrug 88,8%.

### Beispiel 3

Beispiel 1 wurde mit den Ausnahmen wiederholt, dass man bei einen Druck von 39 mbar einstellte, jeweils 15 ml/min Schwefelsäure und Monoethanolamin in die Mischkammer dosierte und den pH-Wert der Quenchflüssigkeit auf 10,8 einstellte. Die Ausbeute betrug 96,3%.

### Beispiel 4

Man verwendete einen Reaktor, der sich von dem in Beispiel 1 beschriebenen lediglich dadurch unterschied, dass die Mischkammer über ein gerades Rohr mit einem Durchmesser von 1 mm mit dem Quenchbehälter verbunden war. Man dosierte 15 ml/min 96%ige Schwefelsäure und 13,9 ml/min Monoethanolamin in die Mischkammer. Die Reaktionstemperatur betrug 280°C, der Druck im Quenchbehälter 45 mbar und der pH-Wert der Quenchflüssigkeit 10,2. Die Ausbeute betrug 95%.

## Patentansprüche

1. Verfahren zur Herstellung von Schwefelsäuremonoestern aus Aminoalkanolen durch Reaktion von Schwefelsäure mit Aminoalkanolen und Entfernen des bei der Reaktion entstehenden Wassers aus dem Reaktionsgemisch, **dadurch gekennzeichnet, dass** man Schwefelsäure und mindestens ein Aminoalkanol mischt, die entstehende heiße Reaktionsmischung unter turbulenter Strömung in einen Behälter leitet und darin mit einem Fluid quencht, wobei zum Quenchen eine wässrige Base verwendet wird.

2. Verfahren nach Anspruch 1, **dadurch gekennzeichnet, dass** man das Verfahren kontinuierlich durchführt.

3. Verfahren nach Anspruch 1 oder 2, **dadurch gekennzeichnet, dass** man Schwefelsäure und Aminoalkanol in einer Mischkammer zusammenführt, die Reaktionsmischung durch ein Rohr mit einem Durchmesser von mindestens 0,5 mm in einen Behälter leitet und darin das Reaktionsgemisch mit einem wässrigen Medium quencht.

4. Verfahren nach einem der Ansprüche 1 bis 3, **dadurch gekennzeichnet, dass** das Reaktionsgemisch aus der Mischkammer durch ein Rohr in den Quenchbehälter geleitet wird, das an der Einmündung in den Quenchbehälter einen Durchmesser aufweist, der um den Faktor 2 bis 1000 größer ist als am Ausgang der Mischkammer.

5. Verfahren nach einem der Ansprüche 1 bis 4, **dadurch gekennzeichnet, dass** man das Reaktionsgemisch mit wässriger Natronlauge quencht.

6. Verfahren nach einem der Ansprüche 1 bis 5, **dadurch gekennzeichnet, dass** man das Reaktionsgemisch mit einer wässrigen Lösung quencht, die ein Alkalimetallsalz eines Schwefelsäuremonoesters eines Aminoalkanols und ein Alkalimetallhydroxid enthält.

7. Verfahren nach einem der Ansprüche 1 bis 6, **dadurch gekennzeichnet, dass** die Temperatur in der Reaktionszone mindestens 220°C beträgt.

8. Verfahren nach einem der Ansprüche 1 bis 7, **dadurch gekennzeichnet, dass** die Temperatur in der Reaktionszone mehr als 250°C beträgt.

9. Verfahren nach einem der Ansprüche 1 bis 8, **dadurch gekennzeichnet, dass** man Schwefelsäure und mindestens ein Aminoalkanol in einer Mischkammer zusammenführt.

10. Verfahren nach einem der Ansprüche 1 bis 9, **dadurch gekennzeichnet, dass** man Schwefelsäure und mindestens ein Aminoalkanol mit Hilfe einer Mehrstoffdüse zusammenführt.

11. Verfahren nach einem der Ansprüche 1 bis 10, **dadurch gekennzeichnet, dass** das Molverhältnis von Schwefelsäure zu Aminoalkanol 1 : 1,5 bis 1 : 0,5 beträgt.

12. Verfahren nach einem der Ansprüche 1 bis 11, **dadurch gekennzeichnet, dass** das Molverhältnis von Schwefelsäure zu Aminoalkanol 1 : 1 bis 1 : 0,9 beträgt.

13. Verfahren nach einem der Ansprüche 1 bis 12, **dadurch gekennzeichnet, dass** die Umsetzung bei vermindertem Druck durchgeführt wird.

14. Verfahren nach einem der Ansprüche 1 bis 13, **dadurch gekennzeichnet, dass** die Umsetzung bei Absolutdrücken von 1 bis 200 mbar durchgeführt wird.

15. Verfahren nach einem der Ansprüche 1 bis 14, **dadurch gekennzeichnet, dass** die Umsetzung bei Absolutdrücken von 15 bis 50 mbar durchgeführt wird.

## Claims

1. A process for the preparation of sulfuric acid monoesters of aminoalkanols by reacting sulfuric acid with aminoalkanols and removing the water forming in the reaction from the reaction mixture, wherein sulfuric acid and at least one aminoalkanol are mixed, and the hot reaction mixture forming is passed with turbulent flow into a container and quenched therein with a fluid, an aqueous base being used for the quenching.

2. The process according to claim 1, which is carried out continuously.

3. The process according to claim 1 or 2, wherein sulfuric acid and aminoalkanol are combined in a mixing chamber, the reaction mixture is passed through a tube having a diameter of at least 0.5 mm into a container and a reaction mixture is quenched therein with an aqueous medium.

4. The process according to any of claims 1 to 3, wherein the reaction mixture is passed from the mixing chamber through a tube into the quench container, which tube, on entering the quench container, has a diameter which is a factor of from 2 to 1000 greater than at the exit of the mixing chamber.

5. The process according to any of claims 1 to 4, wherein the reaction mixture is quenched with aqueous sodium hydroxide solution.

6. The process according to any of claims 1 to 5, wherein the reaction mixture is quenched with an aqueous solution which comprises an alkali metal salt of a sulfuric acid monoester of an aminoalkanol and an alkali metal hydroxide.

7. The process according to any of claims 1 to 6, wherein the temperature in the reaction zone is at least 220°C.

8. The process according to any of claims 1 to 7, wherein the temperature in the reaction zone is more than 250°C.

9. The process according to any of claims 1 to 8, wherein sulfuric acid and at least one aminoalkanol are combined in a mixing chamber.

10. The process according to any of claims 1 to 9, wherein sulfuric acid and at least one aminoalkanol are combined with the aid of a multi-material nozzle.

11. The process according to any of claims 1 to 10, wherein the molar ratio of sulfuric acid to aminoalkanol is from 1:1.5 to 1:0.5.

12. The process according to any of claims 1 to 11, wherein the molar ratio of sulfuric acid to aminoalkanol is from 1:1 to 1:0.9.

13. The process according to any of claims 1 to 12, wherein the reaction is carried out at reduced pressure.

14. The process according to any of claims 1 to 13, wherein the reaction is carried out at absolute pressures of from 1 to 200 mbar.

15. The process according to any of claims 1 to 14, wherein the reaction is carried out at absolute pressures of from 15 to 50 mbar.

## Revendications

1. Procédé de fabrication de monoesters de l'acide sulfurique à partir d'aminoalcanols par réaction d'acide sulfurique avec des aminoalcanols et élimination de l'eau formée lors de la réaction du mélange réactionnel, **caractérisé en ce que** de l'acide sulfurique et au moins un aminoalcool sont mélangés, le mélange réactionnel chaud formé est conduit dans un contenant sous écoulement turbulent, et y est désactivé avec un fluide, une base aqueuse étant utilisée pour la désactivation.

2. Procédé selon la revendication 1, **caractérisé en ce que** le procédé est réalisé en continu.

3. Procédé selon la revendication 1 ou 2, **caractérisé en ce que** l'acide sulfurique et l'aminoalcool sont réunis dans une chambre de mélange, le mélange réactionnel est conduit dans un contenant par un tube d'un diamètre d'au moins 0,5 mm, et le mélange réactionnel y est désactivé avec un milieu aqueux.

4. Procédé selon l'une quelconque des revendications 1 à 3, **caractérisé en ce que** le mélange réactionnel est conduit hors de la chambre de mélange dans le contenant de désactivation par un tube, qui présente au niveau de l'embouchure dans le contenant de désactivation un diamètre qui est supérieur d'un facteur de 2 à 1 000 à celui à la sortie de la chambre de mélange.

5. Procédé selon l'une quelconque des revendications 1 à 4, **caractérisé en ce que** le mélange réactionnel est désactivé avec de la soude caustique aqueuse.

6. Procédé selon l'une quelconque des revendications 1 à 5, **caractérisé en ce que** le mélange réactionnel est désactivé avec une solution aqueuse qui contient un sel de métal alcalin d'un monoester de l'acide sulfurique d'un aminoalcool et d'un hydroxyde de métal alcalins.

7. Procédé selon l'une quelconque des revendications 1 à 6, **caractérisé en ce que** la température dans la chambre de réaction est d'au moins 220 °C.

8. Procédé selon l'une quelconque des revendications 1 à 7, **caractérisé en ce que** la température dans la zone de réaction est supérieure à 250 °C.

9. Procédé selon l'une quelconque des revendications 1 à 8, **caractérisé en ce que** de l'acide sulfurique et au moins un aminoalcool sont réunis dans une chambre de mélange.

10. Procédé selon l'une quelconque des revendications 1 à 9, **caractérisé en ce que** de l'acide sulfurique et au moins un aminoalcool sont réunis à l'aide d'une buse à plusieurs substances.

11. Procédé selon l'une quelconque des revendications 1 à 10, **caractérisé en ce que** le rapport molaire entre l'acide sulfurique et l'aminoalcool est de 1:1,5 à 1:0,5.

12. Procédé selon l'une quelconque des revendications 1 à 11, **caractérisé en ce que** le rapport molaire entre l'acide sulfurique et l'aminoalcool est de 1:1 à 1:0,9.

13. Procédé selon l'une quelconque des revendications 1 à 12, **caractérisé en ce que** la réaction est réalisée à pression réduite.

14. Procédé selon l'une quelconque des revendications 1 à 13, **caractérisé en ce que** la réaction est réalisée à des pressions absolues de 1 à 200 mbar.

15. Procédé selon l'une quelconque des revendications 1 à 14, **caractérisé en ce que** la réaction est réalisée à des pressions absolues de 15 à 50 mbar.
